# EUROPÄISCHE PATENTANMELDUNG

(11) **EP 2 883 857 A1**
(43) Veröffentlichungstag der Anmeldung: **17.06.2015**
(21) Anmeldenummer: 13197159.0
(22) Anmeldetag: 13.12.2013
(51) Int. Cl.: C07C 29/149, C07C 209/16, C07C 51/36, C08G 69/04

(54) **Verfahren zur Herstellung von Adipinsäure oder wenigstens einem Folgeprodukt davon**

(71) Anmelder: BASF SE, 67056 Ludwigshafen (DE)
(72) Erfinder: Die Erfindernennung liegt noch nicht vor
(74) Vertreter: Reitstötter Kinzebach

(57) **Zusammenfassung**

Die vorliegende Erfindung betrifft ein Verfahren zur Herstellung von Adipinsäure oder wenigstens einem Folgeprodukt davon, bei dem man in einer Reaktionszone Muconsäure mit Wasserstoff in Gegenwart wenigstens eines Übergangsmetallkatalysators K und einer wasserhaltigen Flüssigkeit A hydriert, wobei die Muconsäure in der Flüssigkeit A unter den Hydrierungsbedingungen zumindest teilweise unlöslich ist.

## Beschreibung

### HINTERGRUND DER ERFINDUNG

Die vorliegende Erfindung betrifft ein Verfahren zur Herstellung von Adipinsäure oder wenigstens einem Folgeprodukt davon durch katalytische Hydrierung von Muconsäure.

### STAND DER TECHNIK

Adipinsäure ist ein industriell bedeutender Grundstoff und wird insbesondere zur Herstellung von Polyamid 66, das auch als Nylon bezeichnet wird, verwendet. Die Herstellung von Polyamid 66 durch wasserabspaltende Polykondensation von Adipinsäure mit Hexamethylendiamin ist seit langem bekannt. Die bei der Herstellung von Polyamid 66 eingesetzte Adipinsäure wird großtechnisch vor allem durch Oxidation von Cyclohexanol oder Cyclohexanol-/Cyclohexanon-Gemischen, die auch als Anolon bezeichnet werden, mit konzentrierter Salpetersäure hergestellt. Anolon ist durch Oxidation von Cyclohexan mit Luftsauerstoff zugänglich.

Ein weiterer bekannter Ausgangsstoff zu Herstellung von Adipinsäure ist Muconsäure. Muconsäure, systematische Bezeichnung 2,4-Hexadiendicarbonsäure, kann in der cis,cis-, cis,trans- oder trans,trans-Konformation vorliegen. Muconsäure lässt sich beispielsweise durch biochemische Verfahren aus nachwachsenden Rohstoffen wie Glucose oder Lignin gewinnen. Durch vollständige Hydrierung der Kohlenstoff-Kohlenstoff-Doppelbindungen in der Muconsäure erhält man Adipinsäure.

Muconsäure zeichnet sich im Gegensatz zu Adipinsäure durch eine ausgesprochene Schwerlöslichkeit in gängigen Lösungsmitteln wie Wasser und Ethanol aus. So zeigt beispielsweise cis,cis-Muconsäure bei etwa 47 °C eine Löslichkeit in Wasser von etwa 1 g pro 100 g Wasser. Deshalb ist die Umsetzung von Muconsäure im industriellen Maßstab, beispielsweise zu Adipinsäure, generell mit einem erhöhten Aufwand verbunden. Aus diesem Grund finden sich in der Literatur unterschiedliche Vorgehensweisen, um die Muconsäure vor ihrer weiteren Umsetzung, beispielsweise zu Adipinsäure, in eine besser lösliche Form zu überführen. Dazu zählen beispielsweise die Veresterung mit kurzkettigen Alkanolen und die Neutralisation mit Basen, um lösliche Muconsäuresalze zu erhalten.

E. H. Farmer und L. A. Hughes, J. Chem. Soc. 1934, 1929-1938, beschreiben die Herstellung von Adipinsäure ausgehend vom Dinatriumsalz der Muconsäure im wässrigen Medium mit Hydrierkatalysatoren auf Basis von Nickel oder Platin.

J. A. Elvidge et al., J. Chem. Soc. 1950, 2235-2241, beschreiben die Herstellung von cis,trans-Muconsäure und deren Hydrierung zu Adipinsäure in Ethanol in Gegenwart eines Platinkatalysators. Angaben zur Menge des eingesetzten Lösungsmittels und des Katalysators werden nicht gemacht.

US 4,968,612 beschreibt ein Fermentationsverfahren zur Herstellung von Muconsäure sowie die Hydrierung der so erhaltenen Muconsäure zu Adipinsäure. Konkret wird die Muconsäure als 40 gew.-%ige Aufschlämmung in Essigsäure und in Gegenwart eines Palladiumkatalysators auf Kohle zur Reaktion gebracht. Der Wassergehalt der eingesetzten Essigsäure wird nicht angegeben. Nachteilig an dieser Reaktionsweise ist die Verwendung der korrosiven Essigsäure, die den Einsatz hochwertiger korrosionsbeständiger Reaktoren erfordert.

K. M. Draths und J. W. Frost, J. Am. Chem. Soc. 1994, 116, 339-400 und W. Niu et al., Biotechnol. Prog. 2002, 18, 201-211, beschreiben die Herstellung von cis,cis-Muconsäure aus Glucose durch biokatalysierte Synthese mit anschließender Hydrierung der cis,cis-Muconsäure mit Hilfe eines Platinkatalysators zu Adipinsäure. In beiden Fällen wird der pH-Wert der Fermentationsmischung vor der Hydrierung auf oberhalb von 6,3 beziehungsweise auf einen Wert von 7,0 eingestellt. Dabei resultiert eine Lösung von Muconsäuresalzen. Da in beiden Fällen die Fermentationsbrühe zunächst zentrifugiert und nur der Überstand zur Hydrierung eingesetzt wird, wobei dieser nach der Vorgehensweise von Niu et al. zusätzlich vor der Hydrierung zweimal mit Aktivkohle versetzt und filtriert wird, ist davon auszugehen, dass das Hydriergemisch keine feste Muconsäure enthält.

Ein weiteres Verfahren zur Herstellung von Muconsäure aus erneuerbaren Quellen ist beispielsweise in der WO 2010/148080 A2 beschrieben. Nach Beispiel 4 in den Absätzen [0065] und [0066] dieses Dokuments werden 15 g cis,cis-Muconsäure und 150 ml Wasser 15 Minuten lang unter Rückfluss des Wassers erhitzt. Nach Kühlung auf Raumtemperatur, Filtration und Trocknung werden 10,4 g (69 %) cis-trans-Muconsäure erhalten. Die Mutterlauge (4,2 g = 28 Gew.-%, bezogen auf cis,cis-Muconsäure), besteht nicht mehr aus Muconsäure. Sie enthält Lactone und weitere nicht bekannte Reaktionsprodukte.

J. M. Thomas et al., Chem. Commun. 2003, 1126-1127, beschreiben die Hydrierung von Muconsäure zur Adipinsäure mit Hilfe bimetallischer Nanokatalysatoren, die durch spezielle Ankergruppen in den Poren eines mesoporösen Siliciumdioxids eingelagert sind, in reinem Ethanol.

Die WO 2010/141499 beschreibt die Oxidation von Lignin zu Vanillinsäure, deren Decarboxylierung zu 2-Methoxyphenol und weitere Umsetzung zu Catechol und schließlich Oxidation zu Muconsäure sowie die Hydrierung von auf diese Weise erhaltener Muconsäure mit verschiedenen Übergangsmetallkatalysatoren zu Adipinsäure. Das zur Hydrierung eingesetzte Lösungsmittel ist nicht angegeben.

X. She et al., ChemSusChem 2011, 4, 1071-1073, beschreiben die Hydrierung von trans,trans-Muconsäure zur Adipinsäure mit Rhenium-Katalysatoren auf einem Titandioxid-Träger in Lösungsmitteln, ausgewählt unter Methanol, Ethanol, 1-Butanol, Aceton, Toluol und Wasser. Die Hydrierungen werden ausschließlich bei einer erhöhten Temperatur von 120°C durchgeführt. Mit dem verwendeten Katalysator wird in Wasser nur eine geringe Selektivität bezüglich der Adipinsäure erzielt, Hauptprodukt ist die Dihydromuconsäure.

Die aus dem Stand der Technik bekannten Verfahren zur Herstellung von Adipinsäure aus Muconsäure weisen zahlreiche Nachteile auf. Wird die Muconsäure als Salz eingesetzt, so muss dieses zunächst in einem zusätzlichen Schritt hergestellt werden. Weiterhin führt der Einsatz von Muconsäuresalzen zum unerwünschten Anfall von Salz in der Produktmischung, das aufwendig abgetrennt werden muss. Die Hydrierung von Muconsäure in alkoholischen Lösungsmitteln, z. B. Methanol oder Ethanol, führt zu Nebenprodukten wie den Mono- und -diestern von Muconsäure, von Dihydromuconsäure und von Adipinsäure mit den vorgenannten Alkoholen. Damit wird die Ausbeute an Adipinsäure gemindert und die Isolierung der Adipinsäure erschwert. Die Verwendung korrosiver Lösungsmittel wie Essigsäure setzt teure, korrosionsbeständige Reaktionsgefäße voraus. In vielen Lösungsmitteln wie z. B. Ethanol lassen sich nur Muconsäurelösungen niedriger Konzentration erhalten, die für den Fall, dass nur diese Lösungen und keine Suspensionen zur Hydrierung eingesetzt werden, geringe Raum-Zeit-Ausbeuten zur Folge haben. Vielfach werden teure Katalysatoren, etwa auf der Basis von Platin, eingesetzt. Die eingesetzten organischen Lösungsmittel sind vielfach brennbar und/oder ihr Einsatz ist aus Gesundheits- und Umweltaspekten unerwünscht. Die Ausbeuten an Adipinsäure in den im Stand der Technik beschriebenen Verfahren sind vielfach unzureichend.

Der vorliegenden Erfindung liegt die Aufgabe zugrunde, ein Verfahren bereitzustellen, das die Hydrierung von Muconsäure zur Adipinsäure in hoher Ausbeute und Selektivität ermöglicht. Dabei sollen die zuvor beschriebenen Nachteile des Standes der Technik vermieden werden. Insbesondere soll zur Hydrierung möglichst die Muconsäure selbst und nicht ein Muconsäuresalz eingesetzt werden können. Speziell soll das Verfahren einfach, kostengünstig und in einem umweltfreundlichen Reaktionsmedium durchgeführt werden können, ohne die Notwendigkeit einer aufwendigen Abtrennung von Nebenprodukten.

Es wurde nun überraschenderweise gefunden, dass diese Aufgaben gelöst werden, indem man Muconsäure, die zumindest teilweise in fester Form vorliegt, mit Wasserstoff in einer wasserhaltigen Flüssigkeit in Gegenwart eines Übergangsmetallkatalysators hydriert.

### ZUSAMMENFASSUNG DER ERFINDUNG

Die Erfindung betrifft daher ein Verfahren zur Herstellung von Adipinsäure oder wenigstens einem Folgeprodukt davon, bei dem man in einer Reaktionszone Muconsäure mit Wasserstoff in Gegenwart wenigstens eines Übergangsmetallkatalysators M und einer wasserhaltigen Flüssigkeit A hydriert, wobei die Muconsäure in der Flüssigkeit A unter den Hydrierungsbedingungen zumindest teilweise unlöslich ist.

In einer speziellen Ausführungsform wird die Muconsäure in Form einer Suspension zur Hydrierung eingesetzt. Dabei liegt die Muconsäure als teilchenförmige dispergierte Phase in der wasserhaltigen Flüssigkeit vor.

In einer weiteren speziellen Ausführungsform besteht die Flüssigkeit A nur aus Wasser.

Eine weitere spezielle Ausführungsform ist ein Verfahren zur Herstellung von Adipinsäure oder wenigstens einem Folgeprodukt davon, bei dem man in einer Reaktionszone Muconsäure mit Wasserstoff in Gegenwart wenigstens eines Übergangsmetallkatalysators K und einer wasserhaltigen Flüssigkeit A hydriert, wobei die Muconsäure in der Flüssigkeit A unter den Hydrierungsbedingungen zumindest teilweise unlöslich ist, und wobei
- die Flüssigkeit A einen Wasseranteil im Bereich von 95 bis 100 Gew.-%, bezogen auf das Gesamtgewicht der Flüssigkeit A, aufweist,
- der Übergangsmetallkatalysator K metallisches Nickel, metallisches Cobalt, metallisches Rhodium oder eine Mischung aus wenigstens zwei dieser Metalle enthält, und
- man wenigstens einen Teil der Reaktionsmischung aus der Reaktionszone entnimmt, die entnommene Reaktionsmischung einer Auftrennung in eine an Adipinsäure angereicherte Fraktion und eine an Adipinsäure abgereicherte Fraktion unterzieht die an Adipinsäure abgereicherte Fraktion zumindest teilweise in die Reaktionszone zurückführt.

Ein weiterer Gegenstand der Erfindung ist ein Verfahren zur Herstellung von 1,6-Hexandiol, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie zuvor und im Folgenden definiert, wobei Adipinsäure erhalten wird,
b) die in Schritt a) erhaltene Adipinsäure einer Umsetzung mit Wasserstoff in Gegenwart wenigstens eines Hydrierkatalysators unterzieht.

Ein weiterer Gegenstand der Erfindung ist ein Verfahren zur Herstellung von Hexamethylendiamin, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie zuvor und im Folgenden definiert, wobei Adipinsäure erhalten wird,
b) die in Schritt a) erhaltene Adipinsäure einer Umsetzung mit Wasserstoff in Gegenwart wenigstens eines Hydrierkatalysators zu 1,6-Hexandiol unterzieht,
c) das in Schritt b) erhaltene 1,6-Hexandiol einer Aminierung mit Ammoniak in Gegenwart eines Aminierungskatalysators unter Erhalt von Hexamethylendiamin unterzieht.

Ein weiterer Gegenstand der Erfindung ist ein Verfahren zur Herstellung von Polyamid 66, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie zuvor und im Folgenden definiert, wobei Adipinsäure erhalten wird,
b) die in Schritt a) erhaltene Adipinsäure einer Umsetzung mit Wasserstoff in Gegenwart wenigstens eines Hydrierkatalysators zu 1,6-Hexandiol unterzieht,
c) das in Schritt b) erhaltene 1,6-Hexandiol einer Aminierung mit Ammoniak in Gegenwart eines Aminierungskatalysators unter Erhalt von Hexamethylendiamin unterzieht,
d) das in Schritt c) erhaltene Hexamethylendiamin einer Polykondensation mit Adipinsäure unter Erhalt von Polyamid 66 unterzieht.

In einer speziellen Ausführungsform stammt die in Schritt d) eingesetzte Adipinsäure auch zumindest teilweise aus der erfindungsgemäßen Hydrierung von Muconsäure.

### AUSFÜHRUNGSFORMEN DER ERFINDUNG

Im Einzelnen umfasst die Erfindung die folgenden bevorzugten Ausführungsformen:
1. Verfahren zur Herstellung von Adipinsäure oder wenigstens einem Folgeprodukt davon, bei dem man in einer Reaktionszone Muconsäure mit Wasserstoff in Gegenwart wenigstens eines Übergangsmetallkatalysators K und einer wasserhaltigen Flüssigkeit A hydriert, wobei die Muconsäure in der Flüssigkeit A unter den Hydrierungsbedingungen zumindest teilweise unlöslich ist.
2. Verfahren nach Ausführungsform 1, wobei die Reaktionsmischung bei einem Mindestgehalt von 50 Gew.-% Wasser, bezogen auf das Gesamtgewicht der Reaktionsmischung, bei 60 °C einen pH-Wert im Bereich von 1 bis 6, bevorzugt 1 bis 5, besonders bevorzugt 1 bis 4, aufweist.
3. Verfahren nach einer der vorhergehenden Ausführungsformen, wobei die Flüssigkeit A einen Wasseranteil im Bereich von 5 bis 100 Gew.-%, bevorzugt 30 bis 100 Gew.-%, besonders bevorzugt 50 bis 100 Gew.-%, insbesondere 65 bis 100 Gew.-%, speziell 95 bis 100 Gew.-%, bezogen auf das Gesamtgewicht der Flüssigkeit A, aufweist.
4. Verfahren nach einer der vorhergehenden Ausführungsformen, wobei die Muconsäure unter den Hydrierungsbedingungen eine Löslichkeit in der Flüssigkeit A von höchstens 50 g/L aufweist.
5. Verfahren nach einer der vorhergehenden Ausführungsformen, wobei die Muconsäure bei der Hydrierung zumindest teilweise in Form von in der Flüssigkeit A suspendierten Teilchen vorliegt.
6. Verfahren nach einer der vorhergehenden Ausführungsformen, wobei zur Hydrierung eine Flüssigkeit A eingesetzt wird, in der Adipinsäure unter den Reaktionsbedingungen eine Löslichkeit von wenigstens 100 g/L, bevorzugt wenigstens 200 g/L, aufweist.
7. Verfahren einer der vorhergehenden Ausführungsformen, wobei die Muconsäure aus erneuerbaren Quellen stammt, wobei deren Herstellung vorzugsweise durch biokatalytische Synthese aus mindestens einem nachwachsenden Rohstoff erfolgt.
8. Verfahren nach einer der vorhergehenden Ausführungsformen, wobei man Muconsäure einsetzt, die ein ¹⁴C-zu-¹²C-Isotopenverhältnis im Bereich von 0,5×10⁻¹² bis 5×10⁻¹² aufweist.
9. Verfahren nach einer der vorhergehenden Ausführungsformen, wobei der Übergangsmetallkatalysator K ein heterogener Katalysator ist.
10. Verfahren nach einer der vorhergehenden Ausführungsformen, wobei der Übergangsmetallkatalysator K wenigstens ein Übergangsmetall der Gruppen 7, 8, 9, 10 und 11 des Periodensystems (IUPAC), vorzugsweise ausgewählt unter Re, Fe, Ru, Co, Rh, Ir, Ni, Pd, Pt, Cu und Au, insbesondere ausgewählt unter Re, Ru, Co, Rh, Ir, Ni, speziell ausgewählt unter Ni, Co und Rh, aufweist.
11. Verfahren nach einer der vorhergehenden Ausführungsformen, wobei der Übergangsmetallkatalysator K metallisches Nickel, metallisches Cobalt, metallisches Rhodium oder eine Mischung aus wenigstens zwei dieser Metalle enthält.
12. Verfahren nach einer der vorhergehenden Ausführungsformen, wobei der Übergangsmetallkatalysator K ausgewählt ist unter Raney-Nickel, Raney-Cobalt, Rhodium auf einem Trägermaterial und deren Mischungen.
13. Verfahren nach einer der vorhergehenden Ausführungsformen, wobei man wenigstens einen Teil der Reaktionsmischung aus der Reaktionszone entnimmt, die entnommene Reaktionsmischung einer Auftrennung in eine an Adipinsäure angereicherte Fraktion und eine an Adipinsäure abgereicherte Fraktion unterzieht und gegebenenfalls die an Adipinsäure abgereicherte Fraktion zumindest teilweise in die Reaktionszone zurückführt.
14. Verfahren nach Ausführungsform 13, wobei man die Reaktionsmischung einer Kristallisation der Adipinsäure unterzieht und wenigstens einen Teil der Mutterlauge in die Reaktionszone zurückführt.
15. Verfahren zur Herstellung von Adipinsäure oder wenigstens einem Folgeprodukt davon, bei dem man in einer Reaktionszone Muconsäure mit Wasserstoff in Gegenwart wenigstens eines Übergangsmetallkatalysators K und einer wasserhaltigen Flüssigkeit A hydriert, wobei die Muconsäure in der Flüssigkeit A unter den Hydrierungsbedingungen zumindest teilweise unlöslich ist, und wobei
   - die Flüssigkeit A einen Wasseranteil im Bereich von 95 bis 100 Gew.-%, bezogen auf das Gesamtgewicht der Flüssigkeit A, aufweist,
   - der Übergangsmetallkatalysator K metallisches Nickel, metallisches Cobalt, metallisches Rhodium oder eine Mischung aus wenigstens zwei dieser Metalle enthält, und
   - man wenigstens einen Teil der Reaktionsmischung aus der Reaktionszone entnimmt, die entnommene Reaktionsmischung einer Auftrennung in eine an Adipinsäure angereicherte Fraktion und eine an Adipinsäure abgereicherte Fraktion unterzieht die an Adipinsäure abgereicherte Fraktion zumindest teilweise in die Reaktionszone zurückführt.
16. Verfahren nach einer der vorhergehenden Ausführungsformen, wobei man die Hydrierung bei einer Temperatur im Bereich von 20 °C bis 250 °C, bevorzugt 40 °C bis 150 °C, durchführt.
17. Verfahren nach einer der vorhergehenden Ausführungsformen, wobei man die Umsetzung bei einem absoluten Wasserstoffdruck im Bereich von 1 bis 300 bar, bevorzugt 2 bis 100 bar, durchführt.
18. Verfahren nach einer der vorhergehenden Ausführungsformen, wobei man die Hydrierung kontinuierlich durchführt.
19. Verfahren nach einer der vorhergehenden Ausführungsformen, wobei man die Hydrierung in n hintereinander geschalteten Hydrierreaktoren durchführt, wobei n für eine ganze Zahl von wenigstens zwei steht.
20. Verfahren nach Ausführungsform 19, wobei der 1. bis (n-1). Reaktor über einen in einem externen Kreislauf geführten Strom aus der Reaktionszone verfügt.
21. Verfahren nach einer der Ausführungsformen 19 oder 20, wobei man die Hydrierung in dem n. Reaktor adiabatisch durchführt.
22. Verfahren zur Herstellung von 1,6-Hexandiol, bei dem man
   a) Muconsäure einer Hydrierung mit Wasserstoff in Gegenwart einer wasserhaltigen Flüssigkeit A und in Gegenwart wenigstens eines Übergangsmetallkatalysators K unterzieht, wie in einer der Ausführungsformen 1 bis 21 definiert, wobei Adipinsäure erhalten wird;
   b) die in Schritt a) erhaltene Adipinsäure einer Umsetzung mit Wasserstoff in Gegenwart wenigstens eines Hydrierkatalysators unterzieht.
23. Verfahren nach Ausführungsform 22, wobei der in Schritt b) eingesetzte Hydrierkatalysator bezogen auf das Gesamtgewicht des reduzierten Katalysators mindestens 50 Gew.-% Elemente enthält, die ausgewählt sind unter Rhenium, Eisen, Ruthenium, Cobalt, Rhodium, Iridium, Nickel und Kupfer.
24. Verfahren zur Herstellung von Hexamethylendiamin, bei dem man
   a) Muconsäure einer Hydrierung mit Wasserstoff in Gegenwart einer wasserhaltigen Flüssigkeit A und in Gegenwart wenigstens eines Übergangsmetallkatalysators K unterzieht, wie in einer der Ausführungsformen 1 bis 21 definiert, wobei Adipinsäure erhalten wird,
   b) die in Schritt a) erhaltene Adipinsäure einer Umsetzung mit Wasserstoff in Gegenwart wenigstens eines Hydrierkatalysators zu 1,6-Hexandiol unterzieht,
   c) das in Schritt b) erhaltene 1,6-Hexandiol einer Aminierung mit Ammoniak in Gegenwart eines Aminierungskatalysators unter Erhalt von Hexamethylendiamin unterzieht,
25. Verfahren zur Herstellung von Hexamethylendiamin, bei dem man
   a) Muconsäure einer Hydrierung mit Wasserstoff in Gegenwart einer wasserhaltigen Flüssigkeit A und in Gegenwart wenigstens eines Übergangsmetallkatalysators K unterzieht, wie in einer der Ausführungsformen 1 bis 21 definiert, wobei Adipinsäure erhalten wird,
   b) die in Schritt a) erhaltene Adipinsäure einer Umsetzung mit Wasserstoff in Gegenwart wenigstens eines Hydrierkatalysators zu 1,6-Hexandiol unterzieht,
   c) das in Schritt b) erhaltene 1,6-Hexandiol einer Aminierung mit Ammoniak in Gegenwart eines Aminierungskatalysators unter Erhalt von Hexamethylendiamin unterzieht,
   d) das in Schritt c) erhaltene Hexamethylendiamin einer Polykondensation mit Adipinsäure unter Erhalt von Polyamid 66 unterzieht.
26. Verfahren nach Ausführungsform 25, wobei die in Schritt d) eingesetzte Adipinsäure zumindest teilweise nach dem Verfahren nach einer der Ausführungsformen 1 bis 21 herstellt wird.

### BESCHREIBUNG DER ERFINDUNG

Das erfindungsgemäße Verfahren zeichnet sich durch die folgenden Vorteile aus:
- Aus nachwachsenden Rohstoffen hergestellte Muconsäure fällt in der Regel in wässrigen Lösungen an. Nach dem erfindungsgemäßen Verfahren ist eine Hydrierung ohne einen Lösungsmittelwechsel möglich.
- Ein bevorzugtes großtechnisches Aufarbeitungsverfahren ist das Umkristallisieren, so dass auch hier kein Lösungsmittelwechsel erforderlich ist.
- Die bei der Aufarbeitung erhaltenen wässrigen Adipinsäure-haltigen Mutterlaugen können in die Hydrierung zurückgeführt werden.

### Adipinsäure

Bevorzugt wird in dem erfindungsgemäßen Verfahren zur Hydrierung ein Muconsäureeinsatzmaterial eingesetzt, das im Wesentlichen aus Muconsäure besteht. Besonders bevorzugt umfasst das im erfindungsgemäßen Verfahren eingesetzte Muconsäureeinsatzmaterial wenigstens 90 Gew.-%, ganz besonders bevorzugt wenigstens 95 Gew.-%, jeweils bezogen auf das Gesamtgewicht des Muconsäureeinsatzmaterials, an Muconsäure.

Die im erfindungsgemäßen Verfahren einsetzbare Muconsäure kann aus erneuerbaren Quellen stammen, womit natürliche Quellen wie etwa Zucker, z. B. Stärke, Cellulose und Glucose, oder Lignin gemeint sind. Die Herstellung beispielsweise von Muconsäure aus z. B. Stärke, Cellulose, Glucose oder Lignin kann auf alle dem Fachmann bekannten Arten erfolgen, z. B. biokatalytisch. Die biokatalytische Herstellung von cis,cis-Muconsäure durch Fermentationsverfahren ist beispielsweise für Glucose im eingangs zitierten Stand der Technik beschrieben. Die im erfindungsgemäßen Verfahren einsetzbare Muconsäure kann auch aus nicht erneuerbaren Quellen stammen. Geeignet für das erfindungsgemäße Verfahren sind grundsätzlich alle Muconsäuren, unabhängig davon, aus welcher erneuerbaren oder nicht erneuerbaren Quelle sie stammen und auf welchem Syntheseweg sie hergestellt wurden. Bevorzugt stammt die erfindungsgemäß einsetzbare Muconsäure aus erneuerbaren Quellen. Aus erneuerbaren Quellen gewonnene Verbindungen, beispielsweise Muconsäure, weisen ein anderes ¹⁴C-zu-¹²C-Isotopenverhältnis als aus fossilen Quellen wie Erdöl gewonnene Verbindungen auf. Die bevorzugt eingesetzte, aus erneuerbaren Quellen gewonnene Muconsäure weist dementsprechend bevorzugt ein ¹⁴C-zu-¹²C-Isotopenverhältnis im Bereich von 0,5×10⁻¹² bis 5×10⁻¹² auf.

Der Begriff "Muconsäure" umfasst im Sinne der Erfindung die unterschiedlichen Isomere der Muconsäure, nämlich cis,cis-Muconsäure, cis,trans-Muconsäure und trans,trans-Muconsäure in beliebiger Zusammensetzung. Als Einsatzstoffe für das erfindungsgemäße Verfahren sind alle Isomere der Muconsäure geeignet. Bevorzugt besteht die im erfindungsgemäßen Verfahren eingesetzte Muconsäure zu wenigstens 80 Gew.-%, besonders bevorzugt wenigstens 90 Gew.-% aus cis,cis-Muconsäure, bezogen auf das Gesamtgewicht aller in der eingesetzten Muconsäure enthaltenen Muconsäureisomere.

Im Rahmen der Erfindung bezeichnet der Begriff "Muconsäure" ein Einsatzmaterial, das im Wesentlichen aus vollständig protonierter, nicht derivatisierter Muconsäure besteht. Bevorzugt besteht die zur Hydrierung eingesetzte Muconsäure zu mindestens 80 Gew.-%, bevorzugt zu mindestens 95 Gew.-%, insbesondere zu mindestens 99 Gew.-% aus vollständig protonierter, nicht derivatisierter Muconsäure.

Bei der erfindungsgemäßen Hydrierung von Muconsäure können Zwischenprodukte und Nebenprodukte resultieren. Zwischenprodukte sind die der Hydrierung noch zugängigen teilhydrierten Dihydromuconsäuren. Nebenprodukte können beispielsweise durch Wasseranlagerung an eine oder beide Doppelbindungen der eingesetzten Muconsäure und gegebenenfalls nachfolgende Lactonbildung resultieren. Die nach dem erfindungsgemäßen Verfahren erhaltene Adipinsäure kann somit wenigstens ein Zwischen- oder Nebenprodukt enthalten, ausgewählt unter den Isomeren der Dihydromuconsäure, insbesondere 2-Hexendicarbonsäure und 3-Hexendicarbonsäure, den gesättigten und ungesättigten Mono- und Dilactonen (III), (IV) und (V) der Muconsäure sowie Mischungen, die mehrere dieser Zwischen- oder Nebenprodukt enthalten.

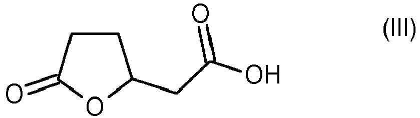

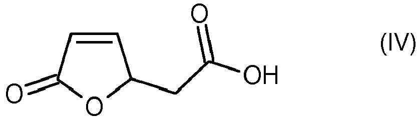

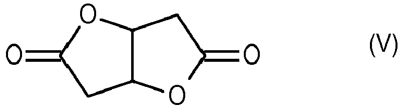

Vorzugsweise enthält das nach dem erfindungsgemäßen Verfahren erhaltene Hydrierungsprodukt höchstens 5 Gew.-%, besonders bevorzugt höchstens 2 Gew.-% an Lactonen der Formeln III bis V, bezogen auf das Gesamtgewicht des Hydrierungsprodukts.

Die wasserhaltige Flüssigkeit A ist eine Substanz oder ein Substanzgemisch, die/das unter den Hydrierungsbedingungen eine flüssige Phase ausbildet. Die Flüssigkeit A verhält sich unter den Hydrierungsbedingungen im Wesentlichen inert, d. h. sie wird im Wesentlichen nicht hydriert. Dementsprechend weist die wasserhaltige Flüssigkeit A vorzugsweise keine ethylenisch ungesättigten Doppelbindungen auf.

Die Flüssigkeit A wird vorzugsweise so gewählt, dass sich das entstehende Verfahrensprodukt Adipinsäure in der Flüssigkeit A unter den Hydrierungsbedingungen mit einem Anteil von wenigstens 1 Gew.-%, bevorzugt wenigstens 10 Gew.-%, besonders bevorzugt wenigstens 20 Gew.-%, bezogen auf das Gesamtgewicht der Flüssigkeit A, löst.

Erfindungsgemäß umfasst die Flüssigkeit A Wasser oder besteht die Flüssigkeit A aus Wasser. Bevorzugt weist die Flüssigkeit A einen Wasseranteil im Bereich von 1 bis 100 Gew.-%, besonders bevorzugt 10 bis 100 Gew.-%, insbesondere 50 bis 100 Gew.-%, jeweils bezogen auf das Gesamtgewicht der Flüssigkeit A, auf. Ganz besonders bevorzugt besteht die Flüssigkeit A ausschließlich aus Wasser, dementsprechend weist die Flüssigkeit A ganz besonders bevorzugt einen Wasseranteil im Bereich von 95 bis 100 Gew.-%, bezogen auf das Gesamtgewicht der Flüssigkeit A, auf.

Bei der wasserhaltigen Flüssigkeit A handelt es sich besonders bevorzugt ausschließlich um Wasser. Die wasserhaltige Flüssigkeit A kann aber auch wenigstens ein unter den Hydrierungsbedingungen flüssig vorliegendes organisches Lösungsmittel enthalten. Bevorzugt sind die organischen Lösungsmittel mit Wasser zumindest teilweise mischbar. Bevorzugt ist das organische Lösungsmittel ausgewählt unter C₁-C₆-Carbonsäuren, linearen und cyclischen, aliphatischen und aromatischen Ethern und Mischungen davon. Beispiele für bevorzugte C₁-C₆-Carbonsäuren sind Ameisensäure, Essigsäure, Propionsäure, Buttersäure, Valeriansäure und Mischungen davon. Beispiele für bevorzugte etherhaltige Lösungsmittel sind Mono- und Di-C₂-C₄-Alkylenglykol-C₁-C₄-alkylether und C₄-C₈-Cycloalkylether, z. B. unsubstituiertes oder C₁-C₄-Alkyl-substituiertes Tetrahydrofuran. Besonders bevorzugte Ether sind Ethylenglykoldimethylether, Ethylenglykoldiethylether, Diethylenglykoldimethylether (Diglyme), Diethylenglykoldi-ethylether, 1,3-Propandioldimethylether, Tetrahydrofuran, 2-Methyltetrahydrofuran, 3-Methyltetrahydrofuran, Diphenylether, Dioxan und Mischungen davon.

Sofern die wasserhaltige Flüssigkeit A wenigstens ein organisches Lösungsmittel enthält, beträgt der Anteil des organischen Lösungsmittels bevorzugt 1 bis 99 Gew.-%, bevorzugt 1 bis 90 Gew.-%, besonders bevorzugt 1 bis 50 Gew.-%, bezogen auf das Gesamtgewicht der Flüssigkeit A. In einer bevorzugten Ausführungsform des erfindungsgemäßen Verfahrens beträgt der Anteil an organischem Lösungsmittel in der Flüssigkeit A weniger als 10 Gew.-%, besonders bevorzugt weniger als 5 Gew.-%, insbesondere weniger als 1 Gew.-%, gezogen auf das Gesamtgewicht der Flüssigkeit A.

Die Muconsäure ist unter den Hydrierungsbedingungen in der Flüssigkeit A zumindest teilweise unlöslich. Bevorzugt handelt es sich bei der Mischung von Muconsäure und Flüssigkeit A um eine Suspension der Muconsäure in der Flüssigkeit A.

Vorzugsweise weist die Reaktionsmischung bei einem Mindestgehalt von 50 Gew.-% Wasser, bezogen auf das Gesamtgewicht der Reaktionsmischung, bei 60 °C einen pH-Wert im Bereich von 1 bis 6, bevorzugt 1 bis 5, besonders bevorzugt 1 bis 4, auf.

Vorzugsweise weist die Flüssigkeit A einen Wasseranteil im Bereich von 5 bis 100 Gew.-%, bevorzugt 30 bis 100 Gew.-%, besonders bevorzugt 50 bis 100 Gew.-%, insbesondere 65 bis 100 Gew.-%, speziell 95 bis 100 Gew.-%, bezogen auf das Gesamtgewicht der Flüssigkeit A, auf.

Bevorzugt weist die Muconsäure unter den Hydrierungsbedingungen eine Löslichkeit in der Flüssigkeit A von vorzugsweise höchstens 80 g/L, besonders bevorzugt höchstens 50 g/L auf.

Bevorzugt wird eine Suspension der Muconsäure, insbesondere von Muconsäure, eingesetzt, worin der Feststoffgehalt der Muconsäure wenigstens 1 Gew.-%, bevorzugt wenigstens 10 Gew.-%, besonders bevorzugt wenigstens 20 Gew.-%, bezogen auf das Gesamtgewicht von Flüssigkeit A und Muconsäure, beträgt. Die Löslichkeit der Muconsäure unter den Hydrierungsbedingungen in der Flüssigkeit A kann vom Fachmann aus Literaturwerten und gegebenenfalls durch einfache Experimente ermittelt werden.

Bevorzugt wird die bei der Hydrierung der Muconsäure erhaltene Adipinsäure gemeinsam mit der Flüssigkeit A aus der Reaktionszone ausgetragen. Dazu ist es vorteilhaft, wenn die Adipinsäure eine gewisse Löslichkeit in der Flüssigkeit A aufweist. Vorzugsweise wird im erfindungsgemäßen Verfahren eine Flüssigkeit A eingesetzt, in der Adipinsäure unter Reaktionsbedingungen eine Löslichkeit von wenigstens 50 g/L, bevorzugt wenigstens 100 g/L, aufweist.

Als Übergangsmetallkatalysatoren K können im erfindungsgemäßen Verfahren grundsätzlich alle dem Fachmann zum Hydrieren von Kohlenstoff-Kohlenstoff-Doppelbindungen bekannten Übergangsmetallkatalysatoren verwendet werden. In der Regel umfasst der Übergangsmetallkatalysator K wenigstens ein Übergangsmetall der Gruppen 7, 8, 9, 10 und 11 des Periodensystems nach IUPAC. Vorzugsweise weist der Übergangs-metallkatalysator K wenigstens ein Übergangsmetall aus der Gruppe Re, Fe, Ru, Co, Rh, Ir, Ni, Pd, Pt, Cu und Au auf. Besonders bevorzugt weist der Übergangsmetallkatalysator K wenigstens ein Übergangsmetall aus der Gruppe Re, Ru, Co, Rh, Ir und Ni auf. Ganz besonders bevorzugt weist der Übergangsmetallkatalysator K wenigstens ein Übergangsmetall aus der Gruppe Ni, Co und Rh auf. Die Übergangsmetallkatalysatoren K umfassen die genannten Übergangsmetalle, insbesondere die als bevorzugt genannten Übergangsmetalle, in der Regel als solche, auf einen Träger aufgebracht, als Fällkatalysatoren, als Raney-Katalysatoren oder als Mischungen davon. Speziell ist der Übergangsmetallkatalysator K ausgewählt unter Raney-Nickel, Raney-Cobalt, Rhodium auf einem Trägermaterial, z. B. Rhodium auf Kohlenstoff, und deren Mischungen.

Als inertes Trägermaterial für die erfindungsgemäßen Übergangsmetallkatalysatoren K können praktisch alle Trägermaterialien des Standes der Technik, wie sie vorteilhaft bei der Herstellung von geträgerten Katalysatoren Verwendung finden, beispielsweise Kohlenstoff, SiO₂ (Quarz), Porzellan, Magnesiumoxid, Zinndioxid, Siliciumcarbid, TiO₂ (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Kohlenstoff, Aluminiumoxid und Siliciumdioxid. Ein besonders bevorzugtes Trägermaterial ist Kohlenstoff. Als Siliciumdioxid-Trägermaterial können Siliciumdioxid-Materialien unterschiedlicher Herkunft und Herstellung, z. B. pyrogen erzeugte Kieselsäuren oder nasschemisch hergestellte Kieselsäuren, wie Kieselgele, Aerogele oder Fällungskieselsäuren, zur Katalysatorherstellung eingesetzt werden (zur Herstellung der verschiedenen SiO₂-Ausgangsmaterialien siehe: W. Büchner, R. Schliebs, G. Winter, K. H. Büchel: Industrielle Anorganische Chemie, 2. Aufl., S. 532-533, VCH Verlagsgesellschaft, Weinheim 1986).

Die Übergangsmetallkatalysatoren K können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren etc. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Übergangsmetallkatalysatoren K können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Die Katalysatorteilchen weisen im Allgemeinen einen Mittelwert des (größten) Durchmessers von 0,5 bis 20 mm, vorzugsweise 1 bis 10 mm, auf. Dazu zählen z. B. Übergangsmetallkatalysatoren K in Form von Tabletten, z. B. mit einem Durchmesser von 1 bis 7 mm, vorzugsweise 2 bis 6 mm, und einer Höhe von 3 bis 5 mm, Ringen mit z. B. 4 bis 7 mm, vorzugsweise 5 bis 7 mm, Außendurchmesser, 2 bis 5 mm Höhe und 2 bis 3 mm Lochdurchmesser, oder Strängen unterschiedlicher Länge eines Durchmessers von z. B. 1,0 bis 5 mm. Derartige Formen können auf an sich bekannte Weise durch Tablettierung, Strangpressen oder Extrusion erhalten werden. Dazu können der Katalysatormasse übliche Hilfsmittel, z. B. Gleitmittel wie Graphit, Polyethylenoxid, Cellulose oder Fettsäuren (wie Stearinsäure) und/oder Formhilfsmittel und Verstärkungsmittel, wie Fasern aus Glas, Asbest oder Siliciumcarbid, zugesetzt werden.

Der Übergangsmetallkatalysator K kann unter den Hydrierungsbedingungen sowohl als homogener als auch als heterogener Katalysator vorliegen. Bevorzugt liegt der Übergangsmetallkatalysator K unter den Hydrierungsbedingungen als heterogener Katalysator vor. Sofern ein heterogener Übergangsmetallkatalysator K verwendet wird, kann dieser beispielsweise auf einem netzförmigen Träger aufgebracht sein. Alternativ oder zusätzlich kann der heterogene Übergangsmetallkatalysator K an der Innenwand eines röhrenförmigen Trägers aufgebracht sein, wobei der röhrenförmige Träger von der Reaktionsmischung durchströmt wird. Da die Muconsäure in der Flüssigkeit A im Wesentlichen als Feststoff vorliegt, sind Ausgestaltungen des Übergangsmetallkatalysators K und/oder des Trägers, auf dem der Übergangsmetallkatalysator K aufgebracht ist, bevorzugt, welche nicht von den Partikeln der Muconsäure, bevorzugt der Muconsäure, verstopft und/oder beschädigt werden. Alternativ oder zusätzlich kann der Übergangsmetallkatalysator K als partikulärer Feststoff eingesetzt werden. In einer bevorzugten Ausführungsform liegt der Übergangsmetallkatalysator K in Form einer Suspension in der Flüssigkeit A vor. Sofern ein flüssiger Reaktionsaustrag aus der Reaktionszone entnommen wird, kann der suspendierte Übergangsmetallkatalysator K durch dem Fachmann bekannte Rückhalteverfahren in der Reaktionszone gehalten werden. Diese Rückhalteverfahren umfassen bevorzugt eine Querstromfiltration, eine Schwerkraftfiltration und/oder eine Filtration mittels wenigstens einer Filterkerze z. B. als Metallsinterfritte.

Bevorzugt erfolgt die Hydrierung bei einer Temperatur im Bereich von 20 °C bis 250 °C, besonders bevorzugt bei einer Temperatur im Bereich von 30 °C bis 200 °C, ganz besonders bevorzugt im Bereich von 40 bis 150 °C.

Die erfindungsgemäße Hydrierung wird bevorzugt bei einem absoluten Wasserstoffdruck im Bereich von 1 bis 300 bar, besonders bevorzugt im Bereich von 1,5 bis 200 bar, ganz besonders bevorzugt im Bereich von 2 bis 100 bar durchgeführt.

Der zur Hydrierung eingesetzte Wasserstoff kann ein oder mehrere inerte Verdünnungsgase, z. B. Stickstoff und/oder Argon enthalten. Bevorzugt wird der zur Hydrierung eingesetzte Wasserstoff im Wesentlichen in reiner Form eingesetzt, d. h. der zur Hydrierung eingesetzte Wasserstoff enthält in der Regel weniger als 10 Gew.-%, bevorzugt weniger als 5 Gew.-%, bezogen auf das Gesamtgewichts des zur Hydrierung eingesetzten Gases, an von Wasserstoff verschiedenen Gasen.

Die mittlere Verweilzeit der Reaktionsmischung in der Reaktionszone liegt in der Regel im Bereich von 0,1 Stunden bis 48 Stunden, bevorzugt im Bereich von 0,2 Stunden bis 24 Stunden, besonders bevorzugt im Bereich von 0,3 Stunden bis 10 Stunden.

Das erfindungsgemäße Verfahren kann je nach Produktionsmenge sinnvoll als Batch-Verfahren, Semi-Batch-Verfahren oder als kontinuierliches Verfahren durchgeführt werden. Bei der Herstellung von technischen Mengen (> 100 to) ist die kontinuierliche Ausführung des erfindungsgemäßen Verfahrens bevorzugt.

Wird das erfindungsgemäße Verfahren als Batch-Verfahren durchgeführt, so geht man in der Regel so vor, dass man Muconsäure, Flüssigkeit A und Übergangsmetallkatalysator K in einem Reaktionsgefäß vorlegt und einmalig Wasserstoff aufpresst. Wird das erfindungsgemäße Verfahren als Semi-Batch-Verfahren durchgeführt, so wird man in der Regel so vorgehen, dass man Muconsäure, Flüssigkeit A und Übergangsmetallkatalysator K in einem Reaktionsgefäß vorlegt und kontinuierlich Wasserstoff nachführt. Nach erfolgter Reaktion wird man in der Regel die erhaltene Adipinsäurelösung aus dem Reaktionsgefäß ausschleusen und gegebenenfalls einer Aufarbeitung, vorzugsweise der gleichen Aufarbeitung wie dem der Reaktionszone entnommenen Teil der Reaktionsmischung beim kontinuierlichen Verfahren, unterziehen. Der Übergangsmetallkatalysator K kann gegebenenfalls durch die genannten Rückhaltevorrichtungen und/oder Rückhalteverfahren abgetrennt und vorzugsweise in wenigstens einem weiteren erfindungsgemäßen Batch- oder Semi-Batch-Verfahren eingesetzt werden.

Das erfindungsgemäße Verfahren wird bevorzugt als kontinuierliches Verfahren durchgeführt. Hierbei wird der Reaktionszone kontinuierlich Muconsäure, Flüssigkeit A, Wasserstoff und gegebenenfalls Übergangsmetallkatalysator K zugeführt und kontinuierlich wenigstens ein Teil der Adipinsäure-haltigen Reaktionsmischung entnommen. Die Muconsäure wird der Reaktionszone bevorzugt ohne Lösungsmittelzusatz als Feststoff oder als Suspension zugeführt. Es versteht sich, dass die Zuführung von Muconsäure, sofern diese ohne Lösungsmittel als Feststoff in die Reaktionszone eingebracht werden soll, räumlich und/oder zeitlich getrennt von der Zuführung der Flüssigkeit A in die Reaktionszone erfolgt. Sofern die Muconsäure als Suspension in die Reaktionszone eingebracht werden soll, ist der Reaktionszone vorzugsweise wenigstens ein Gefäß vorgeschaltet, in dem beispielsweise unter Rühren oder Umpumpen die Suspension der Muconsäure in vorzugsweise der Flüssigkeit A hergestellt wird. Sofern der Übergangsmetallkatalysator K in suspendierter Form eingesetzt wird, kann die Herstellung der Übergangsmetallkatalysatorsuspension zusammen mit der Herstellung der Suspension der Muconsäure in dem wenigstens einen vorgeschalteten Reaktionsgefäß durchgeführt werden.

In einer bevorzugten Ausführungsform des erfindungsgemäßen Verfahrens entnimmt man wenigstens einen Teil der Reaktionsmischung aus der Reaktionszone und unterzieht die entnommene Reaktionsmischung einer Auftrennung in eine an Adipinsäure angereicherte Fraktion und eine an Adipinsäure abgereicherte Fraktion. Geeignet zur Auftrennung in eine Adipinsäure angereicherte Fraktion und eine an Adipinsäure abgereicherte Fraktion sind die dem Fachmann prinzipiell bekannten Trennverfahren, vorzugsweise ausgewählt unter Kristallisationsverfahren, destillativen Verfahren, Adsorptionsverfahren, Ionenaustauschverfahren, Membrantrennverfahren, Extraktionsverfahren oder eine Kombination aus zwei oder mehreren dieser Verfahren. Besonders bevorzugt umfasst die Auftrennung in eine an Adipinsäure angereicherte Fraktion und eine an Adipinsäure abgereicherte Fraktion ein ein- oder mehrstufiges Verfahren zur zumindest teilweisen Kristallisation der Adipinsäure. Die Kristallisation wird bevorzugt bei Temperaturen von 10 bis 80 °C durchgeführt.

Vorzugsweise wird die an Adipinsäure abgereicherte Fraktion zumindest teilweise in die Reaktionszone zurückgeführt. In einer bevorzugten Ausgestaltung unterzieht man die aus der Reaktionszone entnommene Reaktionsmischung zumindest teilweise einer Kristallisation der Adipinsäure und führt wenigstens einen Teil des an Adipinsäure abgereicherten Überstandes (der Mutterlauge) in die Reaktionszone zurück. Die Kristallisation der Adipinsäure kann auch mehrstufig erfolgen.

In einer weiteren Ausgestaltung wird ein homogener, also in der Flüssigkeit im Wesentlichen gelöster, Übergangsmetallkatalysator K verwendet, welcher zusammen mit der Mutterlauge zumindest teilweise in die Reaktionszone zurückgeführt wird. In einer weiteren Ausgestaltung dieser Ausführungsform wird der homogene Übergangsmetallkatalysator K aus dem aus der Reaktionszone entnommene Teil der Reaktionsmischung durch dem Fachmann prinzipiell bekannte Extraktionsverfahren zurückgewonnen. Der zurückgewonnene Teil des homogenen Übergangsmetallkatalysators K kann gegebenenfalls in die Reaktionszone zurückgeführt werden.

Bevorzugt stammt die eingesetzte Muconsäure aus erneuerbaren Quellen. Je nach Reinheitsgrad der eingesetzten Muconsäure kann diese noch Stoffe enthalten, welche für den Übergangsmetallkatalysator K als Katalysatorgift wirken. Es kann sich hierbei beispielsweise um Verbindungen handeln, die Schwefel, Phosphor, Stickstoff und/oder Halogene enthalten. Es kann daher die Notwendigkeit bestehen, den verbrauchten Übergangsmetallkatalysator K kontinuierlich durch unverbrauchten, noch reaktiven Übergangsmetallkatalysator K zu ersetzen. Sofern der Übergangsmetallkatalysator K kontinuierlich durch unverbrauchten, noch reaktiven Übergangsmetallkatalysator K ersetzt werden soll, sind im erfindungsgemäßen Verfahren Übergangsmetallkatalysatoren K bevorzugt, die in der Flüssigkeit A als Suspension vorliegen. Sofern ein suspendierter Übergangsmetallkatalysator K eingesetzt wird und das Verfahren als kontinuierliches Verfahren durchgeführt wird, sind dementsprechend Ausführungsformen des erfindungsgemäßen Verfahrens besonders bevorzugt, in denen kontinuierlich wenigstens ein Teil des suspendierten Übergangsmetallkatalysators K aus der Reaktionszone entfernt wird, z. B. durch Filtrationsverfahren oder teilweise oder vollständige Abreicherung aus einem der Reaktionszone entnommenen Teil der Reaktionsmischung mit oder ohne Rückführung des an Übergangsmetallkatalysator K abgereicherten Teils der entnommenen Reaktionsmischung, und in denen kontinuierlich noch reaktiver Übergangsmetallkatalysator K der Reaktionszone zugeführt wird. Die kontinuierliche Zuführung von Übergangsmetallkatalysator K zur Reaktionszone kann in Form eines Feststoffs oder in Form einer Suspension, bevorzugt einer Suspension in der Flüssigkeit A, erfolgen.

Die Katalysatorbelastung bei kontinuierlicher Fahrweise beträgt vorzugsweise 0,01 bis 100 kg, besonders bevorzugt 0,1 bis 50 kg zu hydrierende Muconsäure pro kg Übergangsmetallkatalysator K.

Das Molverhältnis von Wasserstoff zu Muconsäureverbindung beträgt vorzugsweise 2 : 1 bis 20 : 1, besonders bevorzugt 2 : 1 bis 3 : 1.

In einer speziellen Ausführung des erfindungsgemäßen Verfahrens erfolgt die Hydrierung in n hintereinander (in Reihe) geschalteten Hydrierreaktoren, wobei n für eine ganze Zahl von wenigstens 2 steht. Geeignete Werte für n sind 2, 3, 4, 5, 6, 7, 8, 9 und 10. Bevorzugt steht n für 2 bis 6 und insbesondere für 2 oder 3. In dieser Ausführung erfolgt die Hydrierung bevorzugt kontinuierlich.

Die zur Hydrierung eingesetzten Reaktoren können unabhängig voneinander eine oder mehrere Reaktionszonen innerhalb des Reaktors aufweisen. Bei den Reaktoren kann es sich um gleiche oder verschiedene Reaktoren handeln. Diese können z. B. jeweils gleiche oder verschiedene Vermischungscharakteristiken aufweisen und/oder durch Einbauten ein- oder mehrfach unterteilt sein.

Geeignete druckfeste Reaktoren für die Hydrierung sind dem Fachmann bekannt. Dazu zählen die allgemein üblichen Reaktoren für Gas-Flüssig-Reaktionen, wie z. B. Rohrreaktoren, Rohrbündelreaktoren, Gasumlaufreaktoren, Blasensäulen, Schlaufenapparate, Rührkessel (die auch als Rührkesselkaskaden ausgestaltet sein können), Air-Lift-Reaktoren etc.

Das erfindungsgemäße Verfahren unter Verwendung von heterogenen Übergangsmetallkatalysatoren K kann in Festbett- oder Suspensionsfahrweise durchgeführt werden.

Die Festbettfahrweise kann dabei z. B. in Sumpf- oder in Rieselfahrweise durchgeführt werden. Dabei werden die Übergangsmetallkatalysatoren K vorzugsweise als Formkörper eingesetzt, wie sie zuvor beschrieben sind, z. B. in Form von gepressten Zylindern, Tabletten, Pastillen, Wagenrädern, Ringen, Sternen oder Strangpresslingen, wie Vollsträngen, polylobären Strängen, Hohlsträngen, Wabenkörpern etc.

Für die Hydrierung von feststoffhaltigen Gemischen, wie z. B. von Muconsäuresuspensionen sind Festbettreaktoren nicht geeignet. Sie können im erfindungsgemäßen Verfahren jedoch z. B. als Nachreaktoren eingesetzt werden, in die eine homogene flüssige Phase eingespeist wird.

Bei der Suspensionsfahrweise werden ebenfalls heterogene Katalysatoren eingesetzt. Die heterogenen Katalysatoren werden dabei zumeist in feinverteiltem Zustand eingesetzt und liegen im Reaktionsmedium feinteilig suspendiert vor.

Geeignete heterogene Katalysatoren und Verfahren zu ihrer Herstellung sind die zuvor beschriebenen.

Bei der Hydrierung an einem Festbett wird ein Reaktor eingesetzt, in dessen Innenraum das Festbett angeordnet ist, durch das das Reaktionsmedium strömt. Das Festbett kann dabei aus einer einzigen oder aus mehreren Schüttungen gebildet sein. Jede Schüttung kann dabei eine oder mehrere Zonen aufweisen, wobei wenigstens eine der Zonen ein als Hydrierkatalysator aktives Material enthält. Jede Zone kann dabei ein oder mehrere verschiedene katalytisch aktive Materialien aufweisen und/oder ein oder mehrere verschiedene inerte Materialien aufweisen. Verschiedene Zonen können jeweils gleiche oder verschiedene Zusammensetzungen aufweisen. Es ist auch möglich, mehrere katalytisch aktive Zonen vorzusehen, die beispielsweise durch inerte Schüttungen voneinander getrennt sind. Die einzelnen Zonen können auch unterschiedliche katalytische Aktivität aufweisen. Dazu können verschiedene katalytisch aktive Materialien eingesetzt werden und/oder wenigstens einer der Zonen ein inertes Material beigemischt werden. Das Reaktionsmedium, das durch das Festbett strömt, enthält erfindungsgemäß mindestens eine flüssige Phase, nämlich die inerte Flüssigkeit A. Das Reaktionsmedium kann auch zusätzlich eine gasförmige Phase enthalten.

Als Reaktoren bei der Hydrierung in Suspension kommen insbesondere Schlaufenapparate, wie Strahlschlaufen oder Propellerschlaufen, Rührkessel, die auch als Rührkesselkaskaden ausgestaltet sein können, Blasensäulen oder Air-Lift-Reaktoren zum Einsatz.

Gewünschtenfalls können bei einer Hydriervorrichtung aus n Reaktoren wenigstens zwei der Reaktoren (d. h. 2 bis n der Reaktoren) eine voneinander verschiedene Temperatur aufweisen. In einer speziellen Ausführung wird jeder nachgeschaltete Reaktor mit einer höheren Temperatur betrieben als der vorherige Reaktor. Zusätzlich kann jeder der Reaktoren zwei oder mehr Reaktionszonen mit unterschiedlicher Temperatur aufweisen. So kann beispielsweise in einer zweiten Reaktionszone eine andere, vorzugsweise eine höhere, Temperatur als in der ersten Reaktionszone bzw. in jeder nachfolgenden Reaktionszone eine höhere Temperatur als in einer vorhergehenden Reaktionszone eingestellt werden, z. B. um einen möglichst vollständigen Umsatz bei der Hydrierung zu erzielen.

Gewünschtenfalls können bei einer Hydriervorrichtung aus n Reaktoren wenigstens zwei der Reaktoren (d. h. 2 bis n der Reaktoren) einen voneinander verschiedenen Druck aufweisen. In einer speziellen Ausführung wird jeder nachgeschaltete Reaktor mit einem höheren Druck betrieben als der vorherige Reaktor.

Die Einspeisung des für die Hydrierung benötigten Wasserstoffs kann in den ersten und gegebenenfalls zusätzlich in wenigstens einen weiteren Reaktor erfolgen. Vorzugsweise erfolgt die Einspeisung von Wasserstoff nur in den ersten Reaktor. Die den Reaktoren zugeführte Wasserstoffmenge ergibt sich aus der in der Hydrierreaktion verbrauchten Wasserstoffmenge und der gegebenenfalls mit dem Abgas ausgetragenen Wasserstoffmenge.

Die Einstellung des in dem jeweiligen Reaktor umgesetzten Anteils an Muconsäure kann z. B. über das Reaktorvolumen und/oder die Verweilzeit im Reaktor erfolgen. Der Umsatz im ersten Reaktor, bezogen auf Muconsäure, beträgt vorzugsweise wenigstens 60 %, besonders bevorzugt wenigstens 70 %. Der Gesamtumsatz bei der Hydrierung der Muconsäure, bezogen auf die Muconsäure, beträgt vorzugsweise wenigstens 97 %, besonders bevorzugt wenigstens 98 %, insbesondere wenigstens 99 %.

Zur Abfuhr der bei der exothermen Hydrierung entstehenden Reaktionswärme kann einer oder können mehrere der Reaktoren mit wenigstens einer Kühlvorrichtung versehen sein. In einer speziellen Ausführung ist wenigstens der erste Reaktor mit einer Kühlvorrichtung versehen. Die Abfuhr der Reaktionswärme kann durch Kühlung eines externen Umlaufstroms oder durch interne Kühlung in wenigstens einem der Reaktoren erfolgen. Zur internen Kühlung können die dafür üblichen Vorrichtungen, im Allgemeinen Hohlkörpermodule, wie Field-Rohre, Rohrschlangen, Wärmetauscherplatten, etc. eingesetzt werden. Alternativ kann die Umsetzung auch in einem gekühlten Rohrbündelreaktor erfolgen.

Vorzugsweise erfolgt die Hydrierung in n hintereinander geschalteten Hydrierreaktoren, wobei n für eine ganze Zahl von wenigstens zwei steht, und wobei wenigstens ein Reaktor über einen in einem externen Kreislauf geführten Strom aus der Reaktionszone verfügt (externer Umlaufstrom, Flüssigkeitsumlauf, Schlaufenfahrweise). Vorzugsweise steht n dabei für zwei oder drei.

Bevorzugt erfolgt die Hydrierung in n hintereinander geschalteten Hydrierreaktoren, wobei n vorzugsweise für zwei oder drei steht, und der 1. bis (n-1). Reaktor über einen in einem externen Kreislauf geführten Strom aus der Reaktionszone verfügt.

Bevorzugt erfolgt die Hydrierung in n hintereinander geschalteten Hydrierreaktoren, wobei n vorzugsweise für zwei oder drei steht, und wobei die Umsetzung in dem n. Reaktor (dem letzten von dem zu hydrierenden Reaktionsgemisch durchströmten Reaktor) adiabatisch durchgeführt wird.

Bevorzugt erfolgt die Hydrierung in n hintereinander geschalteten Hydrierreaktoren, wobei n vorzugsweise für zwei oder drei steht, und wobei der n. Reaktor im geraden Durchgang betrieben wird.

Wird ein Reaktor "im geraden Durchgang" betrieben, so soll hier und im Folgenden darunter verstanden werden, dass ein Reaktor ohne Rückführung des Reaktionsproduktes im Sinne der Schlaufenfahrweise betrieben wird. Die Betriebsweise im geraden Durchgang schließt dabei rückvermischende Einbauten und/oder Rühreinrichtungen im Reaktor grundsätzlich nicht aus.

Sofern das in einem der dem ersten Reaktor nachgeschalteten Reaktoren (d. h. dem 2. bis n. Reaktor) hydrierte Reaktionsgemisch nur noch so geringe Anteile an hydrierbarer Muconsäure oder Zwischenprodukten aufweist, dass die bei der Reaktion auftretende Wärmetönung nicht ausreicht, die erwünschte Temperatur im Reaktor zu halten, kann auch eine Erwärmung des Reaktors (oder einzelner Reaktionszonen des zweiten Reaktors) erforderlich sein. Diese kann analog der zuvor beschriebenen Abfuhr der Reaktionswärme durch Erwärmung eines externen Umlaufstroms oder durch interne Erwärmung erfolgen. In einer geeigneten Ausführung kann zur Temperierung eines Reaktors die Reaktionswärme aus wenigstens einem der vorherigen Reaktoren verwendet werden.

Des Weiteren kann die dem Reaktionsgemisch entzogene Reaktionswärme zur Erwärmung der Zuführströme der Reaktoren verwendet werden. Dazu kann z. B. der Zulaufstrom der Muconsäure in den ersten Reaktor zumindest teilweise mit einem externen Umlaufstrom dieses Reaktors gemischt und die vereinigten Ströme dann in den ersten Reaktor geführt werden. Des Weiteren kann bei m = 2 bis n Reaktoren der Zuführstrom aus dem (m-1)-ten Reaktor in dem m-ten Reaktor mit einem Umlaufstrom des m-ten Reaktors gemischt und die vereinigten Ströme dann in den m-ten Reaktor geführt werden. Des Weiteren kann der Zulaufstrom der Muconsäure und/oder ein anderer Zulaufstrom mit Hilfe eines Wärmetauschers erwärmt werden, der mit entzogener Hydrierwärme betrieben wird.

In einer speziellen Ausgestaltung des Verfahrens wird eine Reaktorkaskade aus n in Reihe geschalteten Reaktoren eingesetzt, wobei die Umsetzung in dem n. (n-ten) Reaktor adiabatisch durchgeführt wird. Dieser Begriff wird im Rahmen der vorliegenden Erfindung im technischen und nicht im physiko-chemischen Sinne verstanden. So erfährt das Reaktionsgemisch beim Strömen durch den zweiten Reaktor auf Grund der exothermen Hydrierungsreaktion in der Regel eine Temperaturerhöhung. Unter adiabatischer Reaktionsführung wird eine Vorgehensweise verstanden, bei der die bei der Hydrierung freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem zweiten Reaktor abgeführt, abgesehen von einem Restanteil, der durch natürliche Wärmeleitung und Wärmeabstrahlung vom Reaktor an die Umgebung abgegeben wird. Vorzugsweise wird der n-te Reaktor im geraden Durchgang betrieben.

In einer bevorzugten Ausführung wird zur Hydrierung eine zweistufige Reaktorkaskade eingesetzt, wobei der erste Hydrierreaktor über einen in einem externen Kreislauf geführten Strom aus der Reaktionszone verfügt. In einer speziellen Ausgestaltung des Verfahrens wird eine Reaktorkaskade aus zwei in Reihe geschalteten Reaktoren eingesetzt, wobei die Umsetzung in dem dritten Reaktor adiabatisch durchgeführt wird.

In einer weiteren bevorzugten Ausführung wird zur Hydrierung eine dreistufige Reaktorkaskade eingesetzt, wobei der erste und der zweite Hydrierreaktor über einen in einem externen Kreislauf geführten Strom aus der Reaktionszone verfügen. In einer speziellen Ausgestaltung des Verfahrens wird eine Reaktorkaskade aus drei in Reihe geschalteten Reaktoren eingesetzt, wobei die Umsetzung in dem dritten Reaktor adiabatisch durchgeführt wird. Unter adiabatischer Reaktionsführung wird eine Vorgehensweise verstanden, bei der die bei der Hydrierung freiwerdende Wärmemenge von der Reaktionsmischung im Reaktor aufgenommen und keine Kühlung durch Kühlvorrichtungen angewandt wird.

In einer Ausführungsform kann in wenigstens einem der eingesetzten Reaktoren eine zusätzliche Durchmischung erfolgen. Eine zusätzliche Durchmischung ist insbesondere vorteilhaft, wenn die Hydrierung bei großen Verweilzeiten des Reaktionsgemischs erfolgt. Zur Durchmischung können z. B. die in die Reaktoren eingeführten Ströme eingesetzt werden, indem man diese über geeignete Mischvorrichtungen, wie Düsen, in die jeweiligen Reaktoren einführt. Zur Durchmischung können auch in einem externen Kreislauf geführte Ströme aus dem jeweiligen Reaktor eingesetzt werden.

Zur Vervollständigung der Hydrierung wird dem ersten bis (n-1)ten Reaktor je ein Austrag entnommen, der noch hydrierbare Muconsäure und/oder Zwischenprodukte enthält und in den jeweils nachgeschalteten Hydrierreaktor eingespeist. In einer speziellen Ausführung wird der Austrag in einen ersten und einen zweiten Teilstrom aufgetrennt, wobei der erste Teilstrom als Kreisstrom dem Reaktor, dem er entnommen wurde, wieder zugeführt wird und der zweite Teilstrom dem nachfolgenden Reaktor zugeführt wird. Der Austrag kann gelöste oder gasförmige Anteile an Wasserstoff enthalten. In speziellen Ausführung wird der Austrag aus dem ersten bis (n-1)ten Reaktor einem Phasentrennbehälter zugeführt, in eine flüssige und in eine gasförmige Phase getrennt, die flüssige Phase in den ersten und den zweiten Teilstrom getrennt und die Gasphase zumindest teilweise dem nachfolgenden Reaktor separat zugeführt. In einer alternativen Ausführung wird der Austrag aus dem ersten bis (n-1)ten Reaktor einem Phasentrennbehälter zugeführt und in einen ersten flüssigen an Wasserstoff abgereicherten Teilstrom und einen zweiten an Wasserstoff angereicherten Teilstrom auftrennt. Der erste Teilstrom wird dann als Kreisstrom dem Reaktor wieder zugeführt, dem er entnommen wurde und der zweite Teilstrom dem nachfolgenden Reaktor (als Muconsäureverbindung-M- und wasserstoffhaltiger Feed) zugeführt wird. In einer weiteren alternativen Ausführung erfolgt die Beschickung des zweiten bis n-ten Reaktors mit Wasserstoff nicht über einen dem vorgeschalteten Reaktor entnommenen wasserstoffhaltigen Feed, sondern mit frischem Wasserstoff über eine separate Zuleitung.

Die zuvor beschriebene Verfahrensvariante eignet sich besonders vorteilhaft zur Steuerung der Reaktionstemperatur und des Wärmeübergangs zwischen Reaktionsmedium, begrenzenden Apparatewänden und Umgebung. Eine weitere Möglichkeit zur Steuerung der Wärmebilanz besteht in der Regelung der Eintrittstemperatur des Zulaufs der Muconsäure. So führt eine tiefere Temperatur des eintretenden Zulaufs in der Regel zu einer verbesserten Abführung der Hydrierwärme. Beim Nachlassen der Katalysatoraktivität kann die Eintrittstemperatur höher gewählt werden, um eine höhere Reaktionsgeschwindigkeit zu erreichen und somit die nachlassende Katalysatoraktivität zu kompensieren. Vorteilhafterweise kann so die Standzeit des eingesetzten Übergangsmetallkatalysators K in der Regel verlängert werden.

### 1,6-Hexandiol

Die Erfindung betrifft außerdem ein Verfahren zur Herstellung von 1,6-Hexandiol, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie zuvor definiert, wobei Adipinsäure erhalten wird;
b) die in Schritt a) erhaltene Adipinsäure einer Hydrierung in Gegenwart eines Hydrierkatalysators unter Erhalt von 1,6-Hexandiol unterzieht.

Das erfindungsgemäße Verfahren eignet sich speziell zur Herstellung von 1,6-Hexandiol aus natürlichen Rohstoffquellen. Ein vollständig aus natürlichen Rohstoffquellen hergestelltes 1,6-Hexandiol weist in der Regel ein ¹⁴C-zu-¹²C-Isotopenverhältnis im Bereich von 0,5 x 10⁻¹² bis 5 x 10⁻¹² auf.

Die Hydrierung von Adipinsäure zu 1,6-Hexandiol ist prinzipiell bekannt. Sie erfolgt bevorzugt in flüssiger Phase. Dabei kann die Hydrierung ohne Zusatz eines externen Lösungsmittels oder in Gegenwart eines externen Lösungsmittels erfolgen. Geeignete externe Lösungsmittel sind vorzugsweise ausgewählt unter Wasser, aliphatischen C₁-C₅-Alkoholen (insbesondere ausgewählt aus Methanol, Ethanol, n-Propanol, i-Propanol, n-Butanol, sek.-Butanol, i-Butanol und tert.-Butanol), aliphatischen C₂-C₆-α,ω-Diolen (d. h. Ethylenglykol, 1,3-Propandiol, 1,4-Butandiol, 1,5-Pentandiol oder 1,6-Hexandiol), Ethern (insbesondere ausgewählt aus Tetrahydrofuran, 2-Methyltetrahydrofuran, Diethylether und Methyl-tert.-butylether) und Gemischen daraus. Bevorzugt sind aliphatische C₁-C₅-Alkohole, Wasser und Gemische dieser Lösungsmittel. Besonders bevorzugt sind Methanol, n-Butanol, iso-Butanol, Wasser und Gemische dieser Lösungsmittel. Weiterhin ist es bevorzugt, das Zielprodukt 1,6-Hexandiol als Lösungsmittel zu verwenden. Dabei kann 1,6-Hexandiol für sich allein oder im Gemisch mit Alkoholen und/oder Wasser eingesetzt werden.

Es ist bevorzugt, dass für die katalytische Hydrierung in Schritt b) eine Lösung eingesetzt wird, die 10 bis 60 Gew.-%, besonders bevorzugt 20 bis 50 Gew.-%, ganz besonders bevorzugt 30 bis 50 Gew.-% Adipinsäure enthält.

Vorzugsweise enthält der in Schritt b) eingesetzte Hydrierkatalysator bezogen auf das Gesamtgewicht des reduzierten Katalysators mindestens 50 Gew.-% Elemente, die ausgewählt sind unter Rhenium, Eisen, Ruthenium, Cobalt, Rhodium, Iridium, Nickel und Kupfer.

Für die erfindungsgemäße Hydrierung in Schritt b) werden bevorzugt Katalysatoren verwendet, die mindestens 50 Gew.-% Cobalt sowie mindestens 0,1 Gew.-% Ruthenium und/oder mindestens 0,1 Gew.-% Rhenium, bezogen auf das Gesamtgewicht des reduzierten Katalysators, enthalten. Katalysatoren, die mindestens 50 Gew.-% Cobalt enthalten, können weiterhin insbesondere Phosphorsäure und weitere Übergangsmetalle wie Kupfer, Mangan und/oder Molybdän enthalten.

Die Herstellung eines geeigneten Co-Katalysatorvorläufers ist aus der DE 2 321 101 bekannt. Dieser enthält im nicht reduzierten, kalzinierten Zustand 40 bis 60 Gew.-% Cobalt (berechnet als Co), 13 bis 17 Gew.-% Kupfer (berechnet als Cu), 3 bis 8 Gew.-% Mangan (berechnet als Mn), 0,1 bis 5 Gew.-% Phosphate (berechnet als H₃PO₄) sowie 0,5 bis 5 Gew.-% Molybdän (berechnet als MoO₃). Die EP 636 409 B1 beschreibt die Herstellung weiterer geeigneter Cobalt-Katalysator-Vorläufer, die zu 55 bis 98 Gew.-% aus Cobalt, zu 0,2 bis 15 Gew.-% aus Phosphor, zu 0,2 bis 15 Gew.-% aus Mangan und zu 0,2 bis 15 Gew.-% aus Alkalimetallen (berechnet als Oxid) bestehen. Derartige Katalysator-Vorläufer können durch Behandlung mit Wasserstoff oder Gemischen aus Wasserstoff und Inertgasen wie Stickstoff zu den aktiven, metallisches Cobalt enthaltenden Katalysatoren reduziert werden. Diese Katalysatoren stellen Vollkontakte dar, die ganz überwiegend aus Metall bestehen und keinen Katalysator-Träger enthalten.

Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind beispielsweise in Houben-Weyl, Methoden der Organischen Chemie, Band IV/1c, Georg Thieme Verlag Stuttgart, 1980, S. 45-67 und Beispiele für heterogene Katalysatoren sind beispielsweise in Houben-Weyl, Methoden der Organischen Chemie, Band IV/1c, S. 16 bis 26 beschrieben.

Bevorzugt werden Katalysatoren verwendet, die eines oder mehrere der Elemente aus den Gruppen 3 und 6 bis 11 des Periodensystems der Elemente (IUPAC), bevorzugt Kupfer, Chrom, Molybdän, Mangan, Rhenium, Ruthenium, Cobalt, Nickel oder Palladium, besonders bevorzugt Kupfer, Cobalt oder Rhenium, enthalten.

Die Katalysatoren können allein aus Aktivkomponenten bestehen oder ihre Aktivkomponenten können auf Trägern aufgebracht sein. Als Trägermaterialien eignen sich insbesondere Cr₂O₃, Al₂O₃, SiO₂ und ZrO₂ oder Mischungen daraus.

Besonders bevorzugt sind Katalysatoren, wie sie in EP 0 552 463 A1 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c ≥ 0, d >0, a > b/2, b > a/4, a > c und a > d gilt und x die zur Wahrung der Elektronneutralität pro Formeleinheit erforderliche Anzahl von Sauerstoffionen bezeichnet. Die Herstellung dieser Katalysatoren kann beispielsweise nach den Angaben der EP 552 463 A1 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so dass als besonders bevorzugte Fällungsmittel Alkalicarbonate oder Ammoniumcarbonat einsetzt werden. Es erfolgt eine thermische Behandlung der Zwischenstufen bei Temperaturen im Bereich von 500 °C bis 1000 °C. Die BET-Oberfläche solcher Katalysatoren liegt zwischen 10 und 150 m²/g.

Weiterhin sind als Katalysatoren geeignet, die eine BET-Oberfläche von 50 bis 120 m²/g besitzen, vollständig oder teilweise Kristalle mit Spinellstruktur enthalten und Kupfer in Gestalt von Kupferoxid enthalten.

Auch die WO 2004/085 356 A1 beschreibt für das erfindungsgemäße Verfahren geeignete Kupfer-Katalysatoren, die Kupferoxid, Aluminiumoxid und mindestens eines der Oxide von Lanthan, Wolfram, Molybdän, Titan oder Zirkonium und zusätzlich pulverförmiges metallisches Kupfer, Kupferblättchen, pulverförmigen Zement, Graphit oder ein Gemisch davon enthalten.

Die Hydrierung der Adipinsäure zu 1,6-Hexandiol in Schritt b) erfolgt bevorzugt bei einer Temperatur, die im Bereich von 160 bis 240 °C, besonders bevorzugt 170 bis 230 °C, ganz besonders bevorzugt 170 bis 220 °C liegt.

In einer bevorzugten Ausführungsform des erfindungsgemäßen Verfahrens zur Herstellung von 1,6-Hexandiol aus Muconsäure erfolgt die Hydrierung von Muconsäure in Schritt a) in einem ersten Schlaufenreaktor und die Hydrierung der in Schritt a) erhaltenen Adipinsäure in Schritt b) in einem zweiten Schlaufenreaktor. Vorzugsweise wird das in Schritt b) erhaltene Hydrierungsprodukt in einem nachfolgenden Rohrreaktor, der im geraden Durchgang betrieben wird, nachhydriert.

Unter einem Schlaufenreaktor ist ein Reaktor zu verstehen, bei dem der Reaktorinhalt im Kreis geführt wird. Der Feed kann nach dem Durchströmen des Reaktors in einer Kühlvorrichtung wie beispielsweise einem Wärmetauscher gekühlt, ein Teilstrom des gekühlten Stroms in den Reaktor zurückgeführt und der Reststrom in die nächste Verfahrensstufe geleitet werden. Dabei kann es sich um einen internen oder einen externen Kreislauf handeln. Vorzugsweise kann der externe Kreislauf in einer Kühlvorrichtung, wie beispielsweise einem Wärmetauscher gekühlt werden. Als Wärmetauscher sind Platten-, Rohrbündel- oder Doppelmantel-Wärmetauscher bevorzugt. Durch Ableiten der Reaktionswärme kann der Temperaturanstieg im Reaktor bei der exothermen Hydrierung gut beherrscht werden. Die mittlere Verweilzeit im Schlaufenreaktor beträgt bevorzugt 0,1 bis 10 h, besonders bevorzugt 0,2 bis 4 h.

In einer weiteren bevorzugten Ausführungsform des erfindungsgemäßen Verfahrens finden Schritte a) und b) in dem gleichen Schlaufenreaktor statt, wobei in diesem Schlaufenreaktor zwei Reaktionszonen mit unterschiedlichen Temperaturen vorliegen. Bevorzugt schließt sich in dieser Ausführungsform ein im geraden Durchgang betriebener Rohrreaktor an, in dem das in Schritt b) erhaltene Hydrierungsprodukt nachhydriert wird.

Bevorzugt erfolgen die Hydrierungen in den Schlaufenreaktoren in Sumpf- oder Rieselfahrweise.

Der bei der Hydrierung von Adipinsäure in Wasser als Lösungsmittel erhaltene Reaktionsaustrag stellt eine wässrige 1,6-Hexandiol-Lösung dar. Nach dem Abkühlen und Entspannen des Hydrieraustrags wird das Wasser in der Regel destillativ entfernt, und 1,6-Hexandiol kann in hoher Reinheit (> 97 %) erhalten werden.

### Hexamethylendiamin

Die Erfindung betrifft außerdem ein Verfahren zur Herstellung von Hexamethylendiamin, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie zuvor und im Folgenden definiert, wobei Adipinsäure erhalten wird;
b) die in Schritt a) erhaltene Adipinsäure einer Umsetzung mit Wasserstoff in Gegenwart wenigstens eines Hydrierkatalysators zu 1,6-Hexandiol unterzieht,
c) das in Schritt b) erhaltene 1,6-Hexandiol einer Aminierung mit Ammoniak in Gegenwart eines Aminierungskatalysators unter Erhalt von Hexamethylendiamin unterzieht,

Bezüglich der Verfahrensschritte a) und b) wird auf die vorherigen Ausführungen zu diesen Schritten in vollem Umfang Bezug genommen.

Das in Schritt b) erhaltene 1,6-Hexandiol wird in Schritt c) vorzugsweise mit Ammoniak in Gegenwart des Aminierungskatalysators zu Hexamethylendiamin umgesetzt.

Das in dem erfindungsgemäßen Verfahren synthetisierte Hexamethylendiamin weist in der Regel ein ¹⁴C-zu-¹²C-Isotopenverhältnis im Bereich von 0,5 × 10⁻¹² bis 5 × 10⁻¹² aufweist.

Die erfindungsgemäße Aminierung kann ohne Zufuhr von Wasserstoff, bevorzugt aber unter Zufuhr von Wasserstoff durchgeführt werden.

Als Katalysatoren werden in einer Ausführungsform der Erfindung vorzugsweise überwiegend Cobalt, Silber, Nickel, Kupfer oder Ruthenium oder Gemische dieser Metalle verwendet. Unter "überwiegend" ist dabei zu verstehen, dass eines dieser Metalle zu mehr als 50 Gew.-% im Katalysator (berechnet ohne Träger) enthalten ist. Die Katalysatoren können als Vollkatalysatoren, also ohne Katalysator-Träger oder als Träger-Katalysatoren verwendet werden. Als Träger werden vorzugsweise SiO₂, Al₂O₃, TiO₂, ZrO₂, Aktivkohle, Silikate und/oder Zeolithe verwendet. Die genannten Katalysatoren werden bevorzugt als Festbettkatalysatoren verwendet. Es ist auch möglich, Cobalt, Nickel und/oder Kupfer in Form von Suspensionskatalysatoren vom Raney-Typ einzusetzen.

In einer Ausführungsform der Erfindung erfolgt das Aminieren des 1,6-Hexandiols in homogener Phase und der Katalysator ist ein Komplexkatalysator, der mindestens ein Element ausgewählt aus den Gruppen 8, 9 und 10 des Periodensystems (IUPAC) sowie mindestens einen Donorliganden enthält. Solche Katalysatoren sind beispielsweise aus der WO 2012/119929 A1 bekannt.

Die Aminierung erfolgt bevorzugt bei Temperaturen von 100 bis 250 °C, besonders bevorzugt 120 bis 230 °C, ganz besonders bevorzugt 100 bis 210 °C.

Der Gesamtdruck liegt bevorzugt im Bereich von 5 bis 30 MPa, besonders bevorzugt 7 bis 27 MPa, ganz besonders bevorzugt 10 bis 25 MPa.

Das Molverhältnis von 1,6-Hexandiol zu Ammoniak beträgt bevorzugt 1 zu 30, besonders bevorzugt 1 zu 25, ganz besonders bevorzugt 1 zu 20.

Die Aminierung kann lösungsmittelfrei erfolgen. Bevorzugt wird sie allerdings in Gegenwart wenigstens eines Lösungsmittels durchgeführt. Als Lösungsmittel sind Wasser, Ether oder Gemische dieser Lösungsmittel bevorzugt, wobei Ether besonders bevorzugt ausgewählt sind unter Dioxan, Tetrahydrofuran, 2-Methyltetrahydrofuran, Dioxolan, Dibutylether und Methyl-tert.-butylether.

In einer bevorzugten Ausführungsform des erfindungsgemäßen Verfahrens werden die bei der Hydrierung von Muconsäure anfallenden wässrigen 1,6-Hexandiol-Lösungen ohne Aufarbeitung in dem Aminierungsschritt eingesetzt.

In einer besonders bevorzugten Ausführungsform wird die Aminierung in Gegenwart von Hexamethylenimin als Lösungsmittel oder Hexamethylenimin/Wasser-Gemischen durchgeführt.

Die Lösungsmittelmenge wird vorzugsweise so bemessen, dass 5 bis 80, bevorzugt 10 bis 70, besonders bevorzugt 15 bis 60 gew.-%ige 1,6-Hexandiol-Lösungen entstehen.

Pro Mol 1,6-Hexandiol werden bevorzugt 10 bis 150 Liter, besonders bevorzugt 10 bis 100 Liter Wasserstoff zugeführt.

Der Wasserstoff-Partialdruck liegt bevorzugt im Bereich von 1 bis 40 MPa, besonders bevorzugt 5 bis30 MPa, ganz besonders bevorzugt 10 bis 25 MPa.

In einer Ausführungsform der Erfindung erfolgt die Aminierung von 1,6-Hexandiol mit Ammoniak in einem ersten Teilschritt c1) zu einem Gemisch aus 1-Amino-6-hydroxyhexan und Hexamethylendiamin, welches mehr als 50 Gew.-% 1-Amino-6-hydroxyhexan enthält. Dieses wird in einem Teilschritt c2) zusammen mit Hexamethylendiamin von nicht umgesetztem 1,6-Hexandiol abgetrennt und in einem Teilschritt c3) mit weiterem Ammoniak zu Hexamethylendiamin umgesetzt.

Die Aminierung kann diskontinuierlich oder kontinuierlich, in der Flüssig- oder Gasphase durchgeführt werden, wobei eine kontinuierliche Verfahrensführung bevorzugt ist.

Die Aufarbeitung des noch 1-Amino-6-hydroxyhexan enthaltenden Zielprodukts Hexamethylendiamin erfolgt vorzugsweise destillativ. Da 1-Amino-6-hydroxyhexan und Hexamethylendiamin sehr ähnliche Dampfdrücke besitzen, wird reines Hexamethylendiamin ausgeschleust. Gemische aus 1-Amino-6-hydroxyhexan und Hexamethylendiamin werden in die Destillationsstufe zurückgeführt.

### Polyamid 66

Die Erfindung betrifft außerdem ein Verfahren zur Herstellung von Polyamid 66, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie zuvor und im Folgenden definiert, wobei Adipinsäure erhalten wird,
b) die in Schritt a) erhaltene Adipinsäure einer Umsetzung mit Wasserstoff in Gegenwart wenigstens eines Hydrierkatalysators zu 1,6-Hexandiol unterzieht,
c) das in Schritt b) erhaltene 1,6-Hexandiol einer Aminierung mit Ammoniak in Gegenwart eines Aminierungskatalysators unter Erhalt von Hexamethylendiamin unterzieht,
d) das in Schritt c) erhaltene Hexamethylendiamin einer Polykondensation mit Adipinsäure unter Erhalt von Polyamid 66 unterzieht.

Verfahren zur Herstellung von Polyamid 66 (Nylon, Polyhexamethylenadipinsäureamid) sind dem Fachmann grundsätzlich bekannt. Die Herstellung von Polyamid 66 erfolgt überwiegend durch Polykondensation sogenannter AH-Salzlösungen, d. h. von wässrigen Lösungen, die Adipinsäure und 1,6-Diaminohexan (Hexamethylendiamin) in stöchiometrischen Mengen enthalten. Konventionelle Herstellverfahren für Polyamid 66 sind z. B. in Kunststoffhandbuch, ¾ Technische Thermoplaste: Polyamide, Carl Hanser Verlag, 1998, München, S. 42-71, beschrieben. Es kann insbesondere auch gemäß einer Vorschrift gearbeitet werden, die aus Hans-Georg Elias, Makromoleküle, 4. Auflage, Seiten 796 bis 797, Hüthig-Verlag (1981) bekannt ist. Auf die zuvor genannten Dokumente wird hier in vollem Umfang Bezug genommen.

Die erfindungsgemäße Herstellung von Polyamid 66 umfasst vorzugsweise:
d1) Umsetzen von Adipinsäure und Hexamethylendiamin in einem Molverhältnis von etwa 1:1 zu Hexamethylendiammoniumadipat (AH-Salz), und
d2) Umsetzen des Hexamethylendiammoniumadipats zu Polyamid 66.

Das Umsetzen des Hexamethylendiammoniumadipats zu Polyamid 66 in Schritt d2) erfolgt insbesondere in Gegenwart von Wasser bei einer Temperatur von maximal 280 °C, besonders bevorzugt von maximal 275 °C.

Das erfindungsgemäße Verfahren eignet sich speziell zur teilweisen oder vollständigen Herstellung von Polyamid 66 aus natürlichen Rohstoffquellen. Ein wesentlicher Aspekt der vorliegenden Erfindung ist somit die ökologisch und ökonomisch verbesserte Bereitstellung von Adipinsäure, Hexamethylendiamin und daraus hergestelltem Polyamid 66 aus natürlichen Muconsäurequellen. Ein vollständig aus natürlichen Rohstoffquellen hergestelltes Polyamid 66 weist in der Regel ein ¹⁴C-zu-¹²C-Isotopenverhältnis im Bereich von 0,5 x 10⁻¹² bis 5 x 10⁻¹² auf.

In einer bevorzugten Ausführungsform zur Herstellung von Polyamid 66 wird ein nach dem erfindungsgemäßen Verfahren umfassend die Schritte a) bis c) hergestelltes Hexamethylendiamin mit einer nach Schritte a) des erfindungsgemäßen Verfahrens hergestellten Adipinsäure zu Polyamid 66 polykondensiert.

Die nachfolgenden Beispiele dienen der Erläuterung der Erfindung und sind nicht einschränkend zu verstehen.

### Beispiele:

Verwendete Einsatzstoffe:
cis,cis-Muconsäure (Fa. Aldrich)
Wasser
Wasserstoff
Raney-Nickel
Raney-Cobalt
2 % Rhodium auf Kohlenstoff

Versuchsvorschrift für Beispiele 1 bis 3:

**Tabelle 1**

| | Übergangsmetallkatalysator K | Adipinsäure (Gew.-%) | Muconsäure (Gew.-%) | Lactone III und IV (Gew.-%) | Bislacton V (Gew.-%) |
|---|---|---|---|---|---|
| Beispiel 1 | Raney-Ni | 95 | 0 | 5 | 0 |
| Beispiel 2 | Raney-Co | 95 | 0 | 5 | 0 |
| Beispiel 3 | 2 % Rh/C | 98 | 0 | 2 | 0 |

Man stellte eine Suspension aus 24 g cis,cis-Muconsäure, 56 g Wasser und 1 g des in Tabelle 1 angegebenen Übergangsmetallkatalysators K her und füllte die Suspension in einen 300-mL-Rührautoklaven aus nichtrostendem Stahl 1.4571. Man presste 30 bar Wasserstoff auf, schaltete den Rührer ein (700 U/min) und erhitzte die Mischung über einen Zeitraum von 20 min auf 80 °C. Nachdem die Mischung auf 80 °C aufgeheizt war, erhöhte man den Wasserstoffdruck auf 100 bar und hielt diesen Wasserstoffdruck durch Nachdosieren von Wasserstoff während der Reaktionszeit konstant. Nach 12 Stunden Reaktionszeit wurde die Reaktionsmischung auf etwa 60 °C abgekühlt, der Druck auf Normaldruck verringert und die Reaktionsmischung vom Katalysator abfiltriert. Das Filtrat wurde mittels ¹H-NMR-Spektroskopie untersucht. Mittels ¹H-NMR-Spektroskopie wurden die Ausbeuten an Adipinsäure, die Mengen an nicht umgesetzter cis,cis-Muconsäure sowie die Mengen an gebildeten Nebenprodukten (Verbindungen der Formeln III, IV und V) bestimmt.

### Beispiel 4:

Man suspendierte 15 g 2 % Rh/C-Katalysator in 150 mL Wasser und gab die Suspension in einen 250-mL-Reaktor. Die Suspension wurde gerührt (700 U/min) und auf 80 °C (Reaktorinnentemperatur) aufgeheizt. Diese Temperatur wurde durch eine am Reaktor angebrachte Temperierungsvorrichtung während der Reaktionsdauer konstant gehalten. Man führte kontinuierlich 30 g/Stunde einer 33 gew.-%igen Suspension von cis,cis-Muconsäure in Wasser sowie 50 Normliter/Stunde Wasserstoffgas in den Reaktor. Gleichzeitig führte man kontinuierlich Flüssigkeit und überschüssiges Gas aus dem Reaktor, wobei das Flüssigkeitsvolumen und der Druck (etwa 50 bar) im Reaktor konstant gehalten wurden. Der Reaktor wies vor der Austragsöffnung eine Metallsinterfritte (Porendurchmesser 5 Mikrometer) auf, durch den die suspendierten Katalysatorpartikel und Muconsäurepartikel im Reaktor gehalten wurden. Hinter der Austragsöffnung befand sich die Austragsleitung. Die Austragsleitung wies ein Ventil auf, durch welches man das Gemisch aus ausgetragener Reaktionsmischung und ausgetragenem Gas bei 80 °C auf Umgebungsdruck entspannte. Der pH-Wert der Reaktionslösung, gemessen bei 60 °C lag bei ca. 3. Insgesamt wurden etwa 5 kg der cis,cis-Muconsäuresuspension in dem Reaktor umgesetzt. Die ausgetragene Flüssigkeit wurde mittels ¹H-NMR-Spektroskopie untersucht. Laut ¹H-NMR-Analyse enthielt die ausgetragene Flüssigkeit, nach Entfernung des Wassers, 96 Gew.-% Adipinsäure, 0,5 Gew.-% Muconsäure, 2 Gew.-% Dihydromuconsäure und 1 Gew.-% Lacton I. Anschließend reinigte man die ausgetragene Flüssigkeit durch Kristallisation oder führte eine Nachhydrierung mit anschließender Kristallisation durch.

### Kristallisation:

Die aus dem Reaktor ausgetragene Flüssigkeit wurde durch Kristallisation gereinigt. Hierzu kühlte man 1 kg der ausgetragenen Flüssigkeit von 60 °C langsam auf 20 °C ab, wobei Adipinsäure auskristallisierte. Das Kristallisat wurde abfiltriert und getrocknet. Man erhielt ca. 300 g Adipinsäure mit einer Reinheit von 99,85 %. Man löste die so erhaltene Adipinsäure in 600 g Wasser, erhitzte die Lösung auf 80 °C und kühlte die Lösung langsam auf 20 °C ab, wobei die Adipinsäure auskristallisierte. Das Kristallisat wurde abfiltriert und getrocknet. Man erhielt ca. 270 g Adipinsäure mit einer Reinheit von 99,92 %.

### Nachhydrierung und Kristallisation:

Die aus dem Reaktor ausgetragene Flüssigkeit wurde nachhydriert und durch Kristallisation gereinigt. Es wurden 4 kg der ausgetragenen Flüssigkeit in einem Rieselbettreaktor bei 50 bar Wasserstoffdruck an 200 mg 2 % Rh/C bei 150 °C nachhydriert. Dem Rieselbettreaktor wurden hierbei 50 g/Stunde der ausgetragenen Flüssigkeit und 20 Normliter Wasserstoffgas/Stunde zugeführt. In dem Reaktionsaustrag aus dem Rieselbettreaktor konnten mit ¹H-NMR-Analyse keine ethylenisch ungesättigten Verbindungen mehr nachgewiesen werden. Man erhitzte die aus dem Rieselbettreaktor ausgetragene Flüssigkeit auf 80 °C und kühlte die Flüssigkeit langsam auf 20 °C ab, wobei Adipinsäure auskristallisierte. Das Kristallisat wurde abfiltriert und getrocknet. Man erhielt ca. 310 g Adipinsäure mit einer Reinheit von 99,95 %.

## Patentansprüche

1. Verfahren zur Herstellung von Adipinsäure oder wenigstens einem Folgeprodukt davon, bei dem man in einer Reaktionszone Muconsäure mit Wasserstoff in Gegenwart wenigstens eines Übergangsmetallkatalysators K und einer wasserhaltigen Flüssigkeit A hydriert, wobei die Muconsäure in der Flüssigkeit A unter den Hydrierungsbedingungen zumindest teilweise unlöslich ist.

2. Verfahren nach Anspruch 1, wobei die Reaktionsmischung bei einem Mindestgehalt von 50 Gew.-% Wasser, bezogen auf das Gesamtgewicht der Reaktionsmischung, bei 60 °C einen pH-Wert im Bereich von 1 bis 6, bevorzugt 1 bis 5, besonders bevorzugt 1 bis 4, aufweist.

3. Verfahren nach einem der vorhergehenden Ansprüche, wobei die Flüssigkeit A einen Wasseranteil im Bereich von 5 bis 100 Gew.-%, bevorzugt 30 bis 100 Gew.-%, besonders bevorzugt 50 bis 100 Gew.-%, insbesondere 65 bis 100 Gew.-%, speziell 95 bis 100 Gew.-%, bezogen auf das Gesamtgewicht der Flüssigkeit A, aufweist.

4. Verfahren nach einem der vorhergehenden Ansprüche, wobei die Muconsäure unter den Hydrierungsbedingungen eine Löslichkeit in der Flüssigkeit A von höchstens 50 g/L aufweist.

5. Verfahren nach einem der vorhergehenden Ansprüche, wobei die Muconsäure bei der Hydrierung zumindest teilweise in Form von in der Flüssigkeit A suspendierten Teilchen vorliegt.

6. Verfahren nach einem der vorhergehenden Ansprüche, wobei zur Hydrierung eine Flüssigkeit A eingesetzt wird, in der Adipinsäure unter den Reaktionsbedingungen eine Löslichkeit von wenigstens 100 g/L, bevorzugt wenigstens 200 g/L, aufweist.

7. Verfahren einem der vorhergehenden Ansprüche, wobei die Muconsäure aus erneuerbaren Quellen stammt, wobei deren Herstellung vorzugsweise durch biokatalytische Synthese aus mindestens einem nachwachsenden Rohstoff erfolgt.

8. Verfahren nach einem der vorhergehenden Ansprüche, wobei man Muconsäure einsetzt, die ein ¹⁴C-zu-¹²C-Isotopenverhältnis im Bereich von 0,5×10⁻¹² bis 5×10⁻¹² aufweist.

9. Verfahren nach einem der vorhergehenden Ansprüche, wobei der Übergangsmetallkatalysator K ein heterogener Katalysator ist, der wenigstens ein Übergangsmetall der Gruppen 7, 8, 9, 10 und 11 des Periodensystems (IUPAC), vorzugsweise ausgewählt unter Re, Fe, Ru, Co, Rh, Ir, Ni, Pd, Pt, Cu und Au, insbesondere ausgewählt unter Re, Ru, Co, Rh, Ir, Ni, speziell ausgewählt unter Ni, Co und Rh, aufweist.

10. Verfahren nach einem der vorhergehenden Ansprüche, wobei der Übergangsmetallkatalysator K metallisches Nickel, metallisches Cobalt, metallisches Rhodium oder eine Mischung aus wenigstens zwei dieser Metalle enthält.

11. Verfahren nach einem der vorhergehenden Ansprüche, wobei man wenigstens einen Teil der Reaktionsmischung aus der Reaktionszone entnimmt, die entnommene Reaktionsmischung einer Auftrennung in eine an Adipinsäure angereicherte Fraktion und eine an Adipinsäure abgereicherte Fraktion unterzieht und gegebenenfalls die an Adipinsäure abgereicherte Fraktion zumindest teilweise in die Reaktionszone zurückführt.

12. Verfahren zur Herstellung von Adipinsäure oder wenigstens einem Folgeprodukt davon, bei dem man in einer Reaktionszone Muconsäure mit Wasserstoff in Gegenwart wenigstens eines Übergangsmetallkatalysators K und einer wasserhaltigen Flüssigkeit A hydriert, wobei die Muconsäure in der Flüssigkeit A unter den Hydrierungsbedingungen zumindest teilweise unlöslich ist, und wobei
- die Flüssigkeit A einen Wasseranteil im Bereich von 95 bis 100 Gew.-%, bezogen auf das Gesamtgewicht der Flüssigkeit A, aufweist,
- der Übergangsmetallkatalysator K metallisches Nickel, metallisches Cobalt, metallisches Rhodium oder eine Mischung aus wenigstens zwei dieser Metalle enthält, und
- man wenigstens einen Teil der Reaktionsmischung aus der Reaktionszone entnimmt, die entnommene Reaktionsmischung einer Auftrennung in eine an Adipinsäure angereicherte Fraktion und eine an Adipinsäure abgereicherte Fraktion unterzieht die an Adipinsäure abgereicherte Fraktion zumindest teilweise in die Reaktionszone zurückführt.

13. Verfahren nach einem der vorhergehenden Ansprüche, wobei man die Hydrierung in n hintereinander geschalteten Hydrierreaktoren durchführt, wobei n für eine ganze Zahl von wenigstens zwei steht und wobei der 1. bis (n-1). Reaktor über einen in einem externen Kreislauf geführten Strom aus der Reaktionszone verfügt und man die Hydrierung in dem n. Reaktor adiabatisch durchführt.

14. Verfahren zur Herstellung von 1,6-Hexandiol, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie in einem der Ansprüche 1 bis 21 definiert, wobei Adipinsäure erhalten wird;
b) die in Schritt a) erhaltene Adipinsäure einer Umsetzung mit Wasserstoff in Gegenwart wenigstens eines Hydrierkatalysators unterzieht.

15. Verfahren zur Herstellung von Hexamethylendiamin, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie in einem der Ansprüche 1 bis 21 definiert, wobei Adipinsäure erhalten wird,
b) die in Schritt a) erhaltene Adipinsäure einer Umsetzung mit Wasserstoff in Gegenwart wenigstens eines Hydrierkatalysators zu 1,6-Hexandiol unterzieht,
c) das in Schritt b) erhaltene 1,6-Hexandiol einer Aminierung mit Ammoniak in Gegenwart eines Aminierungskatalysators unter Erhalt von Hexamethylendiamin unterzieht,

16. Verfahren zur Herstellung von Hexamethylendiamin, bei dem man
a) Muconsäure einer Hydrierung mit Wasserstoff in Gegenwart einer wasserhaltigen Flüssigkeit A und in Gegenwart wenigstens eines Übergangsmetallkatalysators K unterzieht, wie in einem der Ansprüche 1 bis 21 definiert, wobei Adipinsäure erhalten wird,
b) die in Schritt a) erhaltene Adipinsäure einer Umsetzung mit Wasserstoff in Gegenwart wenigstens eines Hydrierkatalysators zu 1,6-Hexandiol unterzieht,
c) das in Schritt b) erhaltene 1,6-Hexandiol einer Aminierung mit Ammoniak in Gegenwart eines Aminierungskatalysators unter Erhalt von Hexamethylendiamin unterzieht,
d) das in Schritt c) erhaltene Hexamethylendiamin einer Polykondensation mit Adipinsäure unter Erhalt von Polyamid 66 unterzieht.

17. Verfahren nach Anspruch 16, wobei die in Schritt d) eingesetzte Adipinsäure zumindest teilweise nach dem Verfahren nach einem der Ansprüche 1 bis 21 herstellt wird.
